Europäisches Patentamt

(19) European Patent Office (11) Publication number: **0 049 994**

Office européen des brevets **A2**

(12) # EUROPEAN PATENT APPLICATION

(21) Application number: **81304625.7**

(22) Date of filing: **06.10.81**

(51) Int. Cl.³: **C 12 P 7/06**

(30) Priority: **10.10.80 US 196125**

(43) Date of publication of application:
**21.04.82 Bulletin 82/16**

(84) Designated Contracting States:
**DE FR GB IT SE**

(71) Applicant: BIOSYNTHETICS, INC.
25 East Church Street
Frederick Maryland(US)

(72) Inventor: Lord, Stephen M.
304 Linden Avenue
Frederick Maryland, 21701(US)

(74) Representative: Allden, Thomas Stanley et al,
A.A. THORNTON & CO. Northumberland House 303-306
High Holborn
London WC1V 7LE(GB)

(54) Continuous process for producing alcohol solution.

㊄ A process for the continuous conversion of a starch-containing biomass to an alcohol-containing liquor suitable for distillation or other means of alcohol separation and the concomitant production of distillers grains and distillers solubles, which comprises cooking the starch-containing biomass, slurrying the cooked biomass with water, breaking down the starch molecules in the slurry, pasteurizing the starch slurry at a temperature of from 71°C to 99°C to form starch dissolved and suspended in water, and spent biomass solids, separating the solids from the dissolved and suspended starch solution by countercurrent separation, recovering the separated solids as distillers grains, converting the dissolved and suspended starch solution to a glucose solution, fermenting the glucose to an alcohol-containing liquor and post-fermentation solids, and recovering the post-fermentation solids as distillers solubles.

- 1 -
Continuous process for producing
alcohol solution

The present invention is concerned with a process for producing ethanol from a starch-containing biomass by the solubilization, saccarification and fermentation of the starch and, more particularly, with a process of this kind in which there is a separation of the insoluble and soluble portions of the biomass, with subsequent fermentation of the soluble portion.

The conversion to ethanol of starch-containing biomasses, such as corn, milo, sweet potatoes and Jerusalem artichokes, has been the subject of many studies in recent years as a potential technique for providing a high-octane additive to petrol (gasoline). The cost of the ethanol so produced is usually several percent more than the cost of the petrol to which it is to be added; this is an undesirable economic factor. The unfavourable economics can be reduced, however, when one considers that the cost of ethanol production from starch-containing biomass is the cost of producing the ethanol less the value of any by-products produced.

Two distinct by-products can be recovered in this kind of process. The first consists of the undigested grain protein which is called mash or wet "distillers grains". The second is the "distillers solubles" or thin stillage. The majority of the distillers solubles are not really soluble, but rather are a suspension of fine particles. Both by-products (the grains and solubles) must be collected to obtain the theoretical yield of 7.7 kg of air-dried material per 25.4 kg of corn fermented. The distillers grains accounts for 60% of the 7.7 kg and distillers solubles amount to 40%, depending upon the processing equipment utilized.

The physical and nutritional characteristics of the grains and the solubles should be considered independently. Physically, the grains can be easily separated from the thin stillage by screens, presses or centrifuges. Dry matter can range from 35% to 70%. The thin stillage, however, will contain only 5% to 7% solids. While the grains may be dried to move through normal feed channels, the solubles require a large amount of energy to dry.

The beverage industry presently dries the by-products in several combinations to produce distillers dried grains (DDG), condensed distillers solubles (CDS), distillers dried solubles (DDS), and distillers dried grains plus solubles (DDGS). These products presently move through normal animal feed channels and are used in livestock feeds.

When corn is fermented, the starch is converted to alcohol and carbon dioxide. The starch comprises approximately two-thirds of the corn; therefore, about one-third (7.7 kg) is recovered as by-products from 25.4 kg of corn. Protein, fat, fibre and minerals are concentrated in the by-products. The concentration of protein by starch fermentation converts an energy source (corn) to a protein source (by-product). Maximum nutritional use of the by-products is obtained by feeding them as a protein source rather than as an energy source. The value of the protein in distillers by-products depends upon the characteristics of these proteins, namely the amino acid pattern and protein bypass. As the distillers grains and distillers solubles contain distinctly different proteins, they must be used in different ways to obtain full nutritional value from them.

For swine and poultry, amino acid balance is important. Protein supplements are used in swine and poultry rations to supplement corn grain. Since the protein in corn grain is low in lysine, a high-quality protein such as soybean meal, which is a good source of lysine, is used. Distillers grains are not a good source of supplemental proteins for these rations because its protein, like corn protein, is low in lysine. Distillers solubles is somewhat higher in lysine because it contains some yeast cells and thus will be more valuable than distillers grains as supplemental protein for swine and poultry rations. Distillers grains, on the other hand, have a high bypass value when fed to ruminants. Bypass protein is that protein which escapes digestion in a ruminant and passes, intact, to the small intestine where it is digested. It is this characteristic which makes distillers grains valuable as a protein source for ruminants.

In order to reduce the net cost of manufacturing ethanol by maximizing the value of the by-products, it is important to separate the two types of products: the distillers grains and the distillers solubles. The by-products are similar in that much of the protein is undegraded grain protein. A product similar to distillers grains can be produced if the starch is solubilized and removed from the grains prior to fermentation. Some soluble and fine, suspended protein is also produced and is carried over with the starch. When the starch solution is converted to sugar and fermented, the yeast utilizes some of the soluble protein from the corn and produces yeast cells as part of the alcohol process. These yeast cells can be separated from the fermentation broth and recycled to the beginning of the fermentation in order to increase the yeast titre with the surplus removed for animal feed. The fermentation liquor, after removal of solids, has a very low protein content, which is not worth recovering. Consequently, more effective, higher pressure and higher temperature, cascaded distillation processes can be used to manufacture a fuel-grade alcohol than are commonly used in the beverage industry. Also a portion of the liquid recovered from the alcohol distillation process can be recycled to the cooking process instead of being either discarded or evaporated at a high energy cost.

While these same efficient distillation processes could be utilized on whole mash, that is without solids removal, as is normal practice in the industry, the protein would tend to be degraded by the high temperatures and thus have a lower value. In addition, operating problems tend to occur because of deposition of protein on the internal surfaces of the column. Other alcohol separation systems have also been proposed, such as solvent extraction of the alcohol and absorption of the alcohol with hydrophobic molecular sieves. The use of semi-permeable membranes as a pre-separation alcohol enrichment step has also been proposed. All of these processes would be susceptible to fouling problems and thus require a clear substrate for efficient and continuous operation.

Another means of lowering the overall energy demand of the process lies in recovering the heat used in the cooking process for some useful purpose. In order to do this most effectively, a continuous process is required. A major problem with continuous processes is contamination of the starch solution with microorganisms prior to the addition of yeast. The brewing industry has practised the removal of yeast after fermentation; the yeasts used are those suitable for low temperature fermentation which require very long residence times compared with higher temperature fermentations. In addition, little attention has been paid to the possibility of increasing the rate of fermentation by increasing the yeast concentration because of interest in such things as flavour characteristics which are not important in fuel alcohol production. Yeast removal techniques have typically been decantation or centrifugation. With decantation, some attempt has traditionally been made to recover viable yeast from the centre portion of the fermentation. In other cases, the whole fermentation broth has been chilled so that the yeast will settle to the bottom, the beer has been decanted off, and the yeast then removed in a batch process. In centrifugation, the yeast is all removed at one time and then recycled back to the beginning of the fermentation, perhaps after washing with various chemicals for removal of harmful microorganisms. Very little emphasis in this work has been placed on the energy costs of the processes and the potential loss of alcohol occasioned by the removal of the yeast.

We have now developed a continuous method for the production of a substrate suitable for distillation to fuel-grade alcohol and for the production and recovery of the two types of high protein by-product, that is distillers grains and distillers solubles.

According to the present invention, there is provided a process for the continuous conversion of a starch-containing biomass to an alcohol-containing liquor suitable for distillation or other means of alcohol separation and the concomitant production of distillers grains and distillers solubles, which comprises

cooking the starch-containing biomass,

slurrying the cooked biomass with water,

breaking down the starch molecules in the slurry,

pasteurizing the starch slurry at a temperature of from 71°C to 99°C to form starch dissolved and suspended in water, and spent biomass solids,

separating the solids from the dissolved and suspended starch solution by countercurrent separation,

recovering the separated solids as distillers grains,

converting the dissolved and suspended starch solution to a glucose solution,

fermenting the glucose to an alcohol-containing liquor and post-fermentation solids, and

recovering the post-fermentation solids as distillers solubles.

The process according to the invention is continuous and has lower energy demands than comparable processes. It reduces the severe handling and contamination problems encountered with conventional corn cooking by operating at high temperatures throughout the process, thus reducing the viscosity of the starch solution and pasteurizing it at the same time.

The separation of the dissolved starch from the undissolved protein, fat and fibre, generally termed distillers grains, is achieved by countercurrent separation, and it is thus possible to obtain a high concentration of starch in the liquid with a low loss of starch to the solid portion. After solids removal at relatively high temperatures, the pasteurized liquid is quickly cooled to fermentation temperature. Because of the absence of large quantities of suspended solids, the heat exchangers used may be more efficient than those used for material containing solids and are inherently far less likely to plug. The use of high-efficiency exchangers allows the recovery of this heat for use elsewhere in the process or in related processes.

The starch solution, or wort, going to the fermenter has a low dissolved oxygen concentration because the cooking and solids separation is carried out at high temperature where the solubility of air is lower. The wort is, therefore, suitable for a purely anaerobic fermentation or, by addition of suitable quantities of air, for facultative anaerobic fermentation, such as with yeast.

Advantageously, the fermenters consist of a series of tanks with no internal cooling coils or agitation with the exception of jet nozzles. Cooling and agitation is achieved by a circulation pump on each tank which pumps through a heat exchanger, then back into the tank through nozzles, thus mixing the tank. A portion of the circulating fluid is diverted to the succeeding tank as the feed to it. The minimum number of tanks in series is three, but more stages can be added depending on the alcohol concentration and type of fermenting organism used. A modification of the process uses finishing tanks which serve both to finish the fermentation in a batch mode and as feed tanks for the distillation section. In this case, the yeast removal is on line to the distillation section.

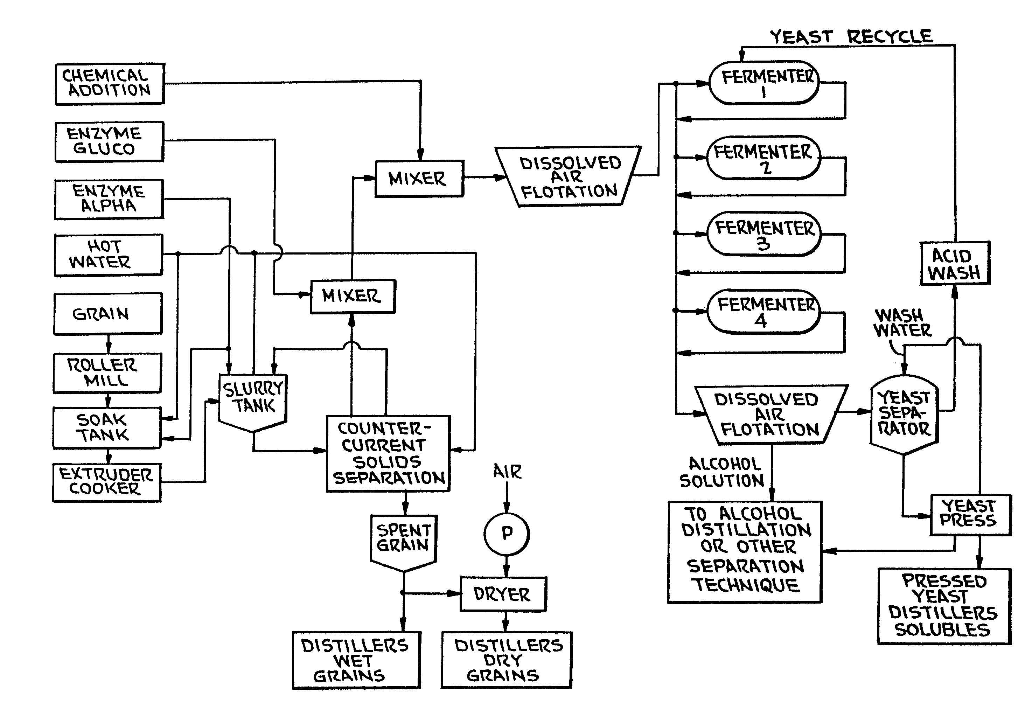

Further features and advantages of the present invention will appear from the following description of a preferred method of carrying out the invention, given by way of example, with reference to the single Figure of the accompanying drawing which is a flow diagram of the process.

As shown in the drawing, a basic feature of the process of the present invention is the continuous nature of all sections of the process. The use of a continuous process allows a more efficient heat integration of the various sections of the process and of the process itself with other processes in an overall, fuel-grade alcohol facility.

According to what is presently believed to be a best method of carrying out the invention, the grain, such as corn, which is shown as a feedstock, is processed through a roller mill, which has lower energy requirements than other means of size reduction. The corn is then fed to a soak tank where it is soaked with hot water and with a high temperature alpha-amylase enzyme. The overall soak time is a function of size reduction in the roller mill, the temperature of the water used, and the concentration of enzyme, as will be apparent to those skilled in this art. For example, the corn is soaked to obtain a moisture content of 35-38%. From the soak tank, the wet, cracked corn is fed into an extruder/cooker. The extruder/cooker is a continuous cooker that can operate satisfactorily on cracked corn, thus reducing the energy requirement for size reduction and providing large particles which permit easier separation in the solids separation section. From the extruder/cooker, the cooked corn goes into a slurry tank where it is mixed with hot water and additional alpha-amylase enzyme. The temperature of the hot water will be sufficient to pasteurize the slurry, that is $71^{\circ}$ to $99^{\circ}C$, preferably $82^{\circ}$ to $99^{\circ}C$, and more preferably $93^{\circ}$ to $99^{\circ}C$. A recycle stream from the countercurrent solids separation is also fed back into this slurry tank. Control of the ratio of recycle to hot water at this point will aid in control of the overall solids concentration, the degree of washing to the starch from the solids, enzyme usage, and fine solids carry over. Thus, in normal practice the ratio of recycle to hot water is preferably from 1 to 10 to all recycle. More preferably it is in a range of 1 to 10 to 10 to 1, and most preferably about 10 to 1, or as much recycle liquor as possible.

The slurry from the tank is fed to the countercurrent solids separation system and the initial liquid separation is recovered for use in the fermenters. The solids-retaining stream of the initial separation is then washed with lower content starch solution to yield the recycle to the slurry tank. The solids are then washed repeatedly in a countercurrent fashion through several stages until, in the final stage, they are washed with hot water, then separated and discharged to a wet solids hopper. In a preferred embodiment, the countercurrent solids separation is carried out in six separate stages, with the flow of hot water against the flow of the slurry solids. Considering the slurry in the slurry tank as upstream, the solids are concentrated in a downstream direction and the concentration of soluble and suspended starch increases in an upstream direction, the liquor containing the soluble and suspended starch from each downstream stage being used to acquire greater starch concentration in the next upstream stage.

The liquid stream from the countercurrent solids separator passes through an initial heat recovery exchanger, a mixer for the addition of glucoamylase enzyme, a further cooler, a mixer for pH adjustment and addition of the yeast food, for example ammonium phosphate, and flocculating agents if it is desired to recover the scum in the dissolved air flotation system where it is aerated and suspended oils and solids are recovered as required. The degree of recovery of these solids is dependent on their composition and the yeast requirements for protein in the fermenters. The degree of aeration is controlled so as to be that suitable for optimum yeast growth in the fermenters.

The wort then goes to the first fermenter, where it is mixed with cooled, recycled fermentation broth and additional yeast recycled from the yeast separation system. In fermenter one, the initial oxygen is utilized for yeast growth and the initial fermentation to alcohol begins. The fermenter temperature is controlled by recycling a portion of the fermentation broth through the heat exchanger. A constant flow of fermentation broth is sent to fermenter two, where the fermentation continues. Again, this fermenter is cooled and agitated, as was fermenter one, by the recycle of fermentation broth through a cooler. A constant flow is sent to fermenter three, where the process is repeated, then to fermenter four which is again cooled and agitated by recirculation through a heat exchanger, but where the fermentation broth and floating solids are drawn off to a yeast separator and then to the distillation system. The yeast that is separated goes to a yeast recovery tank where it is diluted with low alcohol content wash water from the yeast press. Dilution at this point would be about 5 to 10% solids. When multiple yeast recovery tanks are used, the contents of a tank can be allowed to stand, whereupon three layers will form within the tank. The viable yeast is found in the middle layer, which is then decanted and reused in the fermentation process.

After decantation, the remaining contents of the tank are fed to the filter press, where the alcohol-containing solution is first squeezed out, with that portion added back to the distillation column feed tank. The yeast cake is then washed to remove remaining traces of alcohol and this wash water is that which was used to dilute the initial yeast recovered prior to decantation. The filter cake recovered will contain the surplus yeast from the fermentation, plus the carried-over small particles from the corn. It is, thus, very similar to a distillers solubles, except that it will still contain some viable yeast.

The above approach to the preparation of an alcohol-containing liquor suitable for continuous separation, offers a number of significant advantages, such as:

it is designed to remove the two different by-products components, distillers grains and distillers solubles, separately;

it will ease operating problems of the process due to the removal of solids and pasteurization of the starch solution;

it can utilize the possibility of yeast recycle and thus reduce capital costs of fermentation equipment, whether batch or continuous;

it will allow the use of higher efficiency distillation processes, utilizing higher temperatures and pressures (cascaded pressure processes);

it will improve the continuous operation of such columns by reducing the suspended and soluble solids level of the feed to the column; and

it will provide a suitable substrate for most potential low energy separation systems.

CLAIMS:

1.        A process for the continuous conversion of a starch-containing biomass to an alcohol-containing liquor suitable for distillation or other means of alcohol separation and the concomitant production of distillers grains and distillers solubles, which comprises

cooking the starch-containing biomass,

slurrying the cooked biomass with water,

breaking down the starch molecules in the slurry,

pasteurizing the starch slurry at a temperature of from 71°C to 99°C to form starch dissolved and suspended in water, and spent biomass solids,

separating the solids from the dissolved and suspended starch solution by countercurrent separation,

recovering the separated solids as distillers grains,

converting the dissolved and suspended starch solution to a glucose solution,

fermenting the glucose to an alcohol-containing liquor and post-fermentation solids, and

recovering the post-fermentation solids as distillers solubles.

2.        A process according to claim 1, in which yeast food is added to the glucose solution prior to fermentation.

3.        A process according to claim 1 or 2, in which oxygen is dissolved in the glucose solution to provide for yeast growth prior to fermentation.

4.      A process according to any of claims 1 to
3, in which, after fermentation, an oxygen-free gas
is passed through the fermented glucose solution to
aid the formation of floating solids.

5.      A process according to claim 4, in which the
gas is oxygen containing.

6.      A process according to any of claims 1 to
5, in which, after fermentation, yeast is separated
from the fermented glucose solution and the yeast is
recovered as distillers solubles.

7.      A process according to any of claims 1 to
6, in which the starch slurry is pasteurized at a
temperature of from 82°C to 93°C.

8.      A process according to any of claims 1 to
7, in which the countercurrent separation is
accomplished by a flow of hot water in a direction
against the flow of the starch slurry so that the
dissolved and suspended starch moves in the direction
of the hot water flow and the spent solids move against
the hot water flow.

9.      A process according to claim 8, in which the
countercurrent separation is accomplished in a series
of stages in which the concentration of spent solids
in the slurry increases in each stage in the flow of
the slurry and the concentration of the dissolved and
suspended starch decreases in the flow.

10.      A process according to any of claims 1 to
9, in which the starch molecules are broken down by

- 14 -

enzyme action and the dissolved and suspended starch
solution is converted to a glucose solution by
enzyme action.